# EUROPEAN PATENT APPLICATION

(11) **EP 2 174 640 A1**
(43) Date of publication of application: **14.04.2010**
(21) Application number: 08791793.6
(22) Date of filing: 29.07.2008
(51) Int. Cl.: A61K 8/27, A61K 8/29, A61K 33/24, A61K 33/30, A61K 45/00, A61P 17/16, A61Q 17/04, C09K 3/00, G02C 7/10

(54) **PROTECTIVE AGENT AGAINST DAMAGE OF BIOLOGICAL TISSUE CAUSED BY NEAR-INFRARED RAY, AND PRODUCT COMPRISING THE SAME**

(30) Priority: 30.07.2007 JP 2007197592
(71) Applicant: Tanaka, Yohei, Nagano 390-0871 (JP); Matsuo, Kiyoshi, 14-29, Sawamura 1-chome Matsumoto-shi Nagano 390-0877 (JP)
(72) Inventor: Tanaka, Yohei, Nagano 390-0871 (JP); Matsuo, Kiyoshi, 14-29, Sawamura 1-chome Matsumoto-shi Nagano 390-0877 (JP)
(74) Representative: von Kreisler Selting Werner
(86) International application number: PCT/JP2008/063558
(87) International publication number: WO 2009/017104

(57) **Abstract**

The present invention is to provide a protective agent against damage of biological tissue caused by near-infrared rays which is capable of blocking the near-infrared rays from reaching not only skin but also deeper tissue than the skin tissue, preventing damage of such tissue caused by the near-infrared rays and preventing pathogenesis and development of various disease and acceleration of aging both of which are caused by the near-infrared rays, and a product using the protective agent.

The protective agent against damage of biological tissue caused by near-infrared rays comprises a near-infrared ray penetration blocker that absorbs, reflects or scatters the near-infrared rays to prevent the damage of skin and deeper tissue than the skin in a living body caused by exposure with the near-infrared rays.

## Description

### Technical Field

The present invention relates to a protective agent against damage of skin and deeper biological tissue than the skin in a living body, for example a human body, caused by exposure with near-infrared rays in sunrays or artificial rays. The present invention also relates to a product for protecting biological tissue against damage caused by near-infrared rays which comprises the protective agent and is used for protecting biological tissue against morphological variation, functional deterioration, aging and disease.

### Background of the Invention

When normal cells and tissue such as skin are exposed with sunrays especially ultraviolet rays, they are easily damaged as the strong energy of the ultraviolet rays causes erythema, DNA damage and melanin deposition of a biological defense mechanism. When the cells and the tissue are excessively exposed with the sunrays under the scorching sun, melanin cannot fully prevent the ultraviolet rays, and as a result, the ultraviolet rays accelerate development of various dermatological diseases such as cutaneous cancer and aging of the skin. By way of protection against the ultraviolet rays, various products are widely used, for example cosmetics, sunglasses and stick-on films for windows all of which include a protective agent against the ultraviolet rays.

The near-infrared rays, on the other hand, have a property of being well absorbed into hemoglobin and myoglobin, and so they are especially absorbed into tissue with dense vascular component such as skin, muscle, internal organs, brain and bone. By taking advantage of such property, the near-infrared rays are used for a finger arterio authentication system or blood flow measurement of brain. The near-infrared rays also have a property of being well absorbed into water, and so they are applied to beauty treatment for anti-aging where for example the near-infrared rays are irradiated to heat dermis for constricting thereof so that sagging skin can be toned up.

Slight exposure with infrared rays especially with the near-infrared rays has been thought to be harmless. In fact, even considerable exposure with the near-infrared rays has been considered to cause only sensation of warmth on the skin surface in a living body and has no therapeutic problem. Furthermore, influence of the near-infrared rays on deeper biological tissue than the skin in the living body has not been considered.

As a cosmetic application which prevents the sensation of warmth on the skin surface, sunburn on the skin and skin disorder of aging phenomenon such as wrinkles and sag by blocking the infrared rays, the Japanese unexamined application No.62-149613 discloses a product including titanium oxide in the form of thin leaves.

The inventors of the present invention found out that the biological tissue is damaged by the near-infrared rays in spite of its biological defense mechanism. When the normal cells and tissue of skin are excessively exposed with the near-infrared rays in the infrared rays which have approximately 50% of thermal energy in the sunrays under the scorching sun, the biological defense mechanism in the skin works as an acute reaction. When the skin in a living body is exposed with the near-infrared rays, blood vessels in the skin surface layer become dilated, and so hemoglobin is easy to flow into the dilated blood vessels. The near-infrared rays having the property of being well absorbed into the hemoglobin cause the injuries such as rubor and erythema and then make the skin flush. Even if the injury of the subcutaneous tissue caused by the near-infrared rays absorbed into hemoglobin in the skin surface layer can be minimized by the mechanism, the damages still inevitably occur. When the cells and the tissue are further excessively exposed with the near-infrared rays, the near-infrared rays penetrate through the blood vessels more easily. Then exudate, which exudes from a papillary dermis layer, is accumulated in the skin to make vesicle. Although water therein absorbs the near-infrared rays to control the damage of subcutaneous tissue due to the properties that the near-infrared rays are strongly absorbed into water, the damages inevitably occur more.

Furthermore, the inventors found out for the first time that even with relatively slight exposure with the near-infrared rays, the near-infrared rays penetrate through the skin and then cause irreversibly serious damage of not only the skin but also deeper subcutaneous tissue than the skin and human tissue with dense vascular component such as muscle, internal organs, bone and brain, or accelerate aging thereof.

According to these findings, neither insulating the heat caused by the near-infrared rays by blocking thereof on the skin surface nor shielding the ultraviolet rays which expose the skin can sufficiently protect the various deeper tissue than the skin against the damage caused by the near-infrared rays.

### Summary of the Invention

The present invention has been developed to solve the forgoing problems mentioned above and an object of the present invention is to provide a protective agent against damage of biological tissue caused by near-infrared rays which is capable of blocking the near-infrared rays from reaching not only skin but also deeper tissue than the skin tissue, preventing damage of such tissue caused by the near-infrared rays and preventing pathogenesis and development of various disease and acceleration of aging both of which are caused by the near-infrared rays. It is another object of the present invention to provide a product using the protective agent.

The protective agent against damage of biological tissue caused by near-infrared rays developed for accomplishing the foregoing objects comprises a near-infrared ray penetration blocker that absorbs, reflects or scatters the near-infrared rays to prevent the damage of skin and deeper tissue than the skin in a living body caused by exposure with the near-infrared rays.

The protective agent against damage of biological tissue caused by near-infrared rays preferably comprises the near-infrared ray penetration blocker that is at least one selected from the group consisting of metal powder; metal-oxide powder; metal-oxide leaves; metal carbide; metal nitride; metal boride; metal powder coated with metal nitride, metal boride or metal carbide; resin powder coated with metal oxide; mineral powder; ceramics; resin; onium salt compound; azo compound; anthraquinone compound; anfra compound; cyanine compound; metal complex compound; squarylium compound; naphthalocyanine compound; phthalocyanine compound; polyene compound; and polymethine compound.

In the protective agent against damage of biological tissue caused by near-infrared rays, it is preferable that a content of the near-infrared ray penetration blocker ranging from 3 to 45 weight % is included.

In the protective agent against damage of biological tissue caused by near-infrared rays, it is preferable that the near-infrared ray penetration blocker is the metal-oxide powder consisting of titanium oxide powder and zinc oxide powder.

In the protective agent against damage of biological tissue caused by near-infrared rays, it is preferable that a content of the titanium oxide powder ranging from 3 to 25 weight % and a content of the zinc oxide powder ranging from 5 to 20 weight % are included.

A product for protecting biological tissue against damage caused by near-infrared rays comprises;
the protective agent against damage of biological tissue caused by near-infrared rays which is applied, absorbed, adhered, attached or contained to at least a part of a base material, or is contained into the medium in chemicals.

In the product for protecting biological tissue against damage caused by near-infrared rays, it is preferable that a content of the protective agent ranging from 0.0001 to 99.9% by weight to the base material or the chemicals is included.

The product for protecting biological tissue against damage caused by near-infrared rays preferably comprises the base material consisting of paper, fabric, nonwoven fabric, a wood plate, leather, a resin film, a resin sheet, a resin plate, a glass plate and/or a metal plate, and the medium consisting of water, an organic solvent, gel, sol, powder and/or binder.

The product for protecting biological tissue against damage caused by near-infrared rays comprises the base material and is used for clothes, furnishing goods, a protector, accessories, a filter, building materials, external materials, interior materials or decoration materials, or comprises the chemicals and is used for cosmetics, external medicine for skin or coating materials.

The product for protecting biological tissue against damage caused by near-infrared rays is used for preventing aging of the living body or the tissue, morphological variation thereof, functional deterioration thereof, disease pathogenesis, or disease ingravescence.

The present protective agent against damage of biological tissue caused by the near-infrared rays is capable of protecting not only the epidermis of the skin surface but also deeper biological tissue than the skin with dense vascular tissue component such as muscle, internal organs, brain and bone against damage caused by exposure with the near-infrared rays. The protective agent included in the product for protecting biological tissue against damage caused by near-infrared rays enables to certainly show the best effect with less-invasion. Therefore, the product for protecting biological tissue against damage caused by near-infrared rays using such protective agent is effective for prevention of disease caused by long-term exposure with the near-infrared rays and treatment thereof.

The protective agent against damage of biological tissue caused by near-infrared rays and the product for protecting biological tissue against damage caused by the near-infrared rays using the protective agent include no compositional components harmful to human skin and ultraviolet absorbing agents. Therefore, these agent and product can be used repeatedly for human skin for a long period of time.

Furthermore, these agent and product block the near-infrared rays as well as the ultraviolet rays, so these are useful especially for protecting the biological tissue against damage caused by such rays.

### Brief Explanation of the Drawings

Fig. 1 is optical photomicrographs indicating skin, subcutaneous tissue including panniculus carnosus and processus spinosus, and muscle tissue of rats in the absence of near-infrared ray irradiation or the presence of irradiation under different irradiation levels.

Fig. 2 is optical photomicrographs principally indicating bone marrow inside processus spinosus of rat in the absence of the near-infrared ray irradiation or the presence of irradiation under different irradiation levels with or without subcutaneous injection of saline.

Fig. 3 is optical photomicrographs indicating tissue of skin and muscle of rats in the absence or presence of near-infrared ray irradiation.

Fig. 4 is magnified optical photomicrographs indicating the bone marrow inside rat processus spinosus in the absence or presence of near-infrared ray irradiation.

Fig. 5 is a schematic diagram of a method for measuring transmittance and absorptance of the near-infrared rays in the case of using the protective agent against damage of biological tissue caused by near-infrared rays to which the present invention is applied, and a protective agent to which the present invention is not applied.

Fig. 6 is a chart of measurement of transmittance of the near-infrared rays through the protective agent against damage of biological tissue caused by near-infrared rays to which the present invention is applied, and a protective agent to which the present invention is not applied.

Fig. 7 is a chart of measurement of reflectance by back-reference method of the near-infrared rays through the protective agent against damage of biological tissue caused by near-infrared rays to which the present invention is applied, and a protective agent which does not apply the present invention is not applied.

Fig. 8 is a chart of measurement of reflectance by back-dark method of the near-infrared rays through the protective agent against damage of biological tissue caused by near-infrared rays to which the present invention is applied, and a protective agent to which does not apply the present invention is not applied.

Typical reference numerals are;
11: irradiated light, 12(1) and 12(2): a glass slide, 13: a sample, 14: irradiated light, 15: reflected light, 16: reference plate.

### Detailed Explanation of the Invention

Hereunder, the embodiments of the present invention are explained in detail, but the scope of the present invention is not intended to be limited to these embodiments.

One embodiment of the present protective agent against damage of biological tissue caused by near-infrared rays is prepared by kneading a near-infrared ray penetration blocker and if necessary diluents, solvent and dispersing medium.

Examples of the near-infrared ray penetration blocker are;
metal powder such as aluminum powder, gold powder, silver powder, copper powder, platinum powder and stainless-steel powder;
treated metal powder which is coated with or immersed in fatty acids, fats and oils, waxes, aliphatic hydrocarbons, silicone oils, silicone resins, polystyrene resins, polyethylene resins, acrylate resins, epoxy resins, metal hydroxides or a chelating agent on thus metal powder;
another treated metal powder which is coated with metal salts on thus metal powder;
the other treated metal powder illustrated by colored aluminum powder coated with a metal oxide;
metal oxides powder such as rutile-type titanium oxide powder, anatase-type titanium oxide powder, titanium oxide powder including titanium nitride, zinc oxide powder, iron oxide powder, vanadium oxide powder, chromium oxide powder, manganese oxide powder, cobalt oxide powder, gallium oxide powder, zirconium oxide powder, indium oxide powder, tin oxide powder, hafnium oxide powder, ruthenium oxide powder and cerium oxide powder; mixture of the metal oxides powder; ceramics of the sintered metal oxide powder; doped metal thereof; or semimetal oxide powder such as glass powder;
metal oxide leaves such as titanium oxide leaves and zirconium oxide leaves;
metal carbide whose metal is illustrated by aluminum, zirconium, tungsten, niobium, silicon, boron and titanium;
metal nitride whose metal is illustrated by aluminum, chromium, silicon, zirconium, titanium, tantalum, hafnium, vanadium and niobium; treated metal nitride which is coated with metal oxide on thus metal nitride;
metal boride whose metal is illustrated by tantalum, tungsten, zirconium, chromium, niobium, lanthanum, praseodymium, neodymium, cerium, yttrium, titanium, or molybdenum;
resin powder coated with the above-mentioned metal oxide,
mineral powder illustrated by a mineral such as talc, sericite, kaolin and muscovite white mica; treated mineral powder which is coated with aluminum on thus mineral;
ceramics;
a resin illustrated by polyamide resin applied with microarticulated zirconium oxide;
onium salt compound illustrated by a diimmonium compound, an aminium compound, an indolinium compound and a quinolinium compound;
an azo compound;
an anthraquinone compound;
an anfra compound;
a cyanine compound;
a metal complex compound illustrated by a dithiol complex compound such as a dithiol-nickel complex compound, and a nickel complex compound;
a squarylium compound;
a phthalocyanine compound; and
a polyene compound or a polymethine compound.

The exemplified near-infrared ray penetration blocker may be used solely or plurally with mixing.

Either the near-infrared ray penetration blocker is consisted of inorganic compounds or organic compounds, the near-infrared ray penetration blocker has a property of blocking the near-infrared ray transmissivity.

Especially, it is furthermore preferable that the near-infrared ray penetration blocker include metal oxide powder. Examples of the metal-oxide powder are titanium oxide powder, zinc oxide powder, indium oxide powder and so on, and it may be metal oxide powder doped with metal such as indium oxide doped with tin as a metal-oxide semiconductor. When the near-infrared ray penetration blocker includes the metal oxide powder, the metal oxide powder is preferably consisted of 3 to 25 weight % of titanium oxide powder and 5 to 20 weight % of zinc oxide powder. To improve an blocking efficiency against the near-infrared rays, the metal oxide powder such as the titanium oxide powder and/or the zinc oxide powder may be specially coated by a coating layer having thickness of 0.1 to 100 microns, preferably 1 to 10 microns. With such special coating procedure, a coating layer is formed by 1 weight % or less of resin illustrated by silicone resin and polyol etc., a coating oxide layer is formed by dipping into mineral salt including aluminum, silicone, titanium or zinc, or hydroxide thereof, or if necessary then added with acids or alkalis for neutralization and sintered. The metal oxide powder such as the titanium oxide powder and the zinc oxide powder may have an average particle size of 0.1 to 400 microns, and it can be in a form of grains or thin leaves. The titanium oxide and the zinc oxide of grade listed in The Japanese Pharmacopoeia can be used.

As the content of the metal oxide powder such as the titanium oxide powder and the zinc oxide powder increase, the effect of blocking the near-infrared rays increased. If the content thereof is less than the before-mentioned range, the blocking effect thereof becomes insufficient, and so it cannot achieve the sufficient effect as the external medication for the skin of the protective agent against damage of biological tissue caused by near-infrared rays. On the other hands, if the content exceeds the above-mentioned range, the external medication for the skin cannot be prepared due to component separation and poor mixing. The zinc oxide forms a layer by bonding to protein of the skin inducing anti-inflammatory potency and skin protective potency, but on the other hand, the zinc oxide has a property of absorbing exudate and thus drying the skin. Therefore, the content of the zinc oxide powder is preferably controlled to be less in case of applying the external medication for the skin to a person with dry skin.

One of the embodiments of the present invention of the product for protecting biological tissue from damage caused by near-infrared rays comprise a base material having the protective agent on at least a part thereof. The protective agent included in such product absorbs, reflects or scatters the near-infrared rays to prevent damage of skin and deeper tissue than the skin in a living body, morphological variation thereof and functional deterioration thereof so that aging of the human tissue is prevented. The base material can be selected from paper, fabric, nonwoven fabric, a wood plate, leather, a resin film, a resin sheet, a resin plate, a glass plate and a metal plate, preferably selected from the resin film, the resin sheet and the glass plate. The protective agent may be kneaded, dissolved, dispersed, applied, adsorbed, adhered, attached or contained to the base material. By using the base material, a filter can be formed. Detailed examples of such product are a pair of glasses or sunglasses both of which comprise the protective agent against damage by near-infrared rays on their lens parts in the base material. Other examples of the product are clothing such as a hat or a cap, clothes, a scarf, an inner cloth, an underwear and gloves; furnishing goods such as an umbrella and a parasol; protectors such as a helmet, a goggle and a mask; accessories such as a hairpiece; building materials such as a window glass; external materials or interior materials of for example a car and a train; decoration materials such as a curtain.

Another embodiment of the product for protecting biological tissue from damage caused by near-infrared rays is the chemicals including the protective agent against damage of biological tissue by near-infrared rays in the medium. Examples of the embodiment are cosmetics or external medications. Examples of the medium are water, an organic solvent, sol, gel, and binder. The protective agent against damage of biological tissue by near-infrared rays is included by blending, adding or mixing into the medium.

Examples of the cosmetic and the external preparation for skin are a shampoo, a rinse, a hair conditioner, a hair treatment, a hair spray, a hair wax, a hair gel, water grease, a set lotion, a color lotion, a hair tonic, a hair liquid, pomade, a hair cream, a hair blow, a coating for split hair, a hair oil, hair coloring products, a hair dye, a hair manicure, a hair restorer, a facial cleanser, cleansing foam, cleaning powder, facial cleansing powder, a cleansing lotion, a cleaning gel, a cleansing cream, cleansing milk, a cleansing oil, a cleansing pack, a face lotion, emollient, an astringent lotion, a beauty wash, pharmaceutical cosmetics, an emulsion, an emollient lotion, a moisture lotion, a milky lotion, a nourishing lotion, nourishing milk, a sun protect, a sun protecter, ultraviolet care milk, a sunscreen, a makeup lotion, a makeup cream, a hand lotion, a hand cream, a body lotion, a body cream, an emollient cream, a moisture cream, a nourishing cream, a base cream, a pre-makeup cream, a sunscreen cream, a suntan cream, a depilatory cream, a deodorant cream, a shaving cream, soap, toilet soap, medicated soap, liquid soap, shaving soap, synthesized soap, a pack, a masque, an essence, a moisturizing essence, a skin-lightening essence, an essence for protecting from ultraviolet,a beauty essence, a skincare, face powder, powder, foundations, lipsticks, a lip balm, a lip gloss, rouges, an eyeliner, mascara, an eye shadow, an eyebrow pencil, nail enamel, an enamel remover, a nail treatment, deodorant cosmetics, an insect repellent spray, an ointment, a plaster, lotions, and paint.

The chemicals such as the cosmetics and the external medication may include at least one selected from the group consisting of a humectant, a smoothing agent, a surfactant, a polymer, a thickener, gelling agent, an emulsifying agent, a solvent, an oleum, a propellant, an antioxidant, a reducing agent, an oxidizing agent, an antiseptic agent, an antimicrobial agent, a chelating agent, a pH adjuster, acids, alkalis, powder, an inorganic salt, an ultraviolet absorbent, an ultraviolet ray shielding agent, a stabilizing agent, a skin-lightening agent, vitamins, vitamin derivates, antiphlogistics, an anti-Inflammatory agent, a hair growing agent, a circulation promoter, a stimulant, hormones, an anti-wrinkle agent, an anti-aging agent, a contractile agent, an astringent, an algefacient, a calefacient, a wound healing accelerator, a torpent, an analgesic agent, a cellar stimulant, an antibacterial, a plant extract, an animal extract, a microorganism extract, an antipruritic agent, a cuticle remover, a cuticle dissolving agent, a peeling agent, an antiperspirant, refrigerant, enzymes, nucleic acids, perfumes, stains, colorant, dye, pigment, and an antifoaming agent.

The chemicals such as the cosmetics and the external medication may include common additive agents such as a diluting agent, vehicle and solvent medium, for example, an olive oil, glycerin, a vegetable fat, an animal fat, wax, paraffin, starch, a long-chain fatty acid and salt thereof, a cellulose derivative, polyethylene glycol, silicone, bentonite, silicic acid, talc, water and so on.

The formulation of chemicals such as the cosmetic and the external medication for skin may be aerosols, liquids, ointments, suspensions, emulsions, and lotions. The form thereof may be liquid, pasty, creamy, gelatinous or misty.

Concretely, the chemicals such as the cosmetics and the external medication, which are prepared by blending, include;
as powdery ingredients, the near-infrared ray penetration blocker (3-45 weight%) such as titanium oxide powder (3-25 weight%) and zinc oxide powder (5-20 weight%),
as oily ingredients, the emulsifying agents (1 weight% respectively) such as dimethicones illustrated by cetyl dimethicone copolyol or PEG-10 dimethicone in which PEG-10 is polyethylene glycol having 10 of average repeating units, and the antifoaming agent (1 weight%) such as phenyl trimethicone,
as the other oily ingredients, silicone oil (5-42 %) such as cyclopentanesiloxanes (15.5 weight%) and trimethylsiloxysilicate (5 weight%), alkyl benzoate whose alkyl has 12-15 carbons and also acts for the emollient agent, various oleum (1-5 weight%) such as the vegetable oil, squalane, and fatty acid alkyl esters,
as the aqueous ingredients, phenoxyethanol (0.3-1 weight%), glycerin (0-10 weight%), 1,3-butylene glycol (0-10 weight%), various plant extract or animal extract (maximum 1 weight%), various vitamins (maximum 1 weight%), salts (moderately) such as the acids, the alkalis and the pH adjuster, and
as the solvent, purified water(the rest thereof) such as ion-exchanged water, reverse osmosis water and distilled water.

The ingredients except for the near-infrared ray penetration blocker may be properly increased or decreased according to dispersibility into the protective agent or applicableness onto the skin.

The near-infrared rays penetrate through not only the skin and the subcutaneous tissue but also bone and brain, so long-term exposure with the near-infrared rays may cause brain atrophy which leads dementia, demyelination of nerve and serious damage with denaturalization of tissue with dense vessel component, gene damage and tumorigenic transformation. The external medication for the skin is able to protect the tissue with dense vascular component against damage caused exposure by the near-infrared rays. Examples of the damage are degenerative disorder such as cerebrovascular disease, brain blood vessel type dementia, Alzheimer type dementia, demyelinating disease and Parkinson's disease; ophthalmic disorder such as retinopathy; dermatological disease; vascular pathology such as angioma and angiosarcoma; collagen disease such as systemic lupus erythematosus (SLE), scleroderma, polymyositis and cutaneous myositis; blood disease, hematopoietic disease; infection disease; immune disease; allergic disease; disease of tract, abdomen wall and peritoneum; disease of liver, cholecyst, pancreas and spleen; disease of heart and vascular channel; disorder of internal secretion, metabolism and nutrition; disease of kidney and organa urinaria; disorder of apparatus respiratorius, chest wall, mediastinum; disorder of nerve, mental and locomotorium.

Moreover, the external medication for skin has a preventive effect especially against the above-mentioned diseases.

Therefore, the external medication for the skin has medicinal potency as a preventive medicine or therapeutic medicine for the diseases that develop or become worse when the biological defense mechanism as a trigger caused by the exposure with the near-infrared rays works.

The external medication for the skin is used for prophylactic method and therapeutic method for the various diseases mentioned above.

Hereunder, the results of medical investigation conducted to accomplish the present invention are explained in detail.

Because near-infrared rays have properties of exceedingly sufficient penetration and absorption for skin, they are capable of penetrating through the skin and heating dermis or subcutaneous tissue. It was investigated that the near-infrared rays are capable of causing irreversible damage to tissue in a living body, especially the tissue with dense vascular component such as dermis and deeper tissue than the skin illustrated by muscle, internal organs, brain and bone.

Near-infrared ray irradiation was performed with a near-infrared ray therapeutic apparatus: Titan, which is available from Cutera Inc. and used in anti-aging treatment of heating dermis to constrict thereof for improving wrinkles and sag. This therapeutic apparatus is capable of irradiating the near-infrared rays having wavelength of 1.1 to 1.8 microns that is well absorbed by water.

For histological studies, rats having each back being shaved were divided into 7 groups. Three of these groups were subjected to weak irradiation of the near-infrared rays under output power of 20 J/cm² and then further subjected to 1, 2 or 3 rounds of irradiation respectively. Three of the remaining groups were subjected to strong irradiation of the near-infrared rays under output power of 40 J/cm² and then further subjected to 1, 2 or 3 rounds of irradiation respectively. The interval of the irradiation was set to be 1 week.

Under anaesthesia, the near-infrared rays were irradiated to the back of the rats. Samples including skin, subcutaneous tissue, panniculus carnosus and processus spinosus were taken from the back immediately, 1 week, 2 weeks, 1 month, 2 months and 3 months post final-shot. The obtained samples were immediately fixed in formalin and absolute ethanol.

Specimens were evaluated by hematoxylin-eosin staining (i.e. HE staining), AZAN staining and transferase-mediated dUTP nick-end labeling (TUNEL) technique. In HE staining, cell nuclei are stained blue purple, while cytoplasm, connective tissue and erythrocyte are stained red. In AZAN staining, collagen fibers and muscle fibers are stained different colors respectively. In TUNEL labeling technique, DNA fragmentation is demonstrated by histochemical identification of apotosis.

The remaining group was subjected to a control group without irradiation of the near-infrared rays.

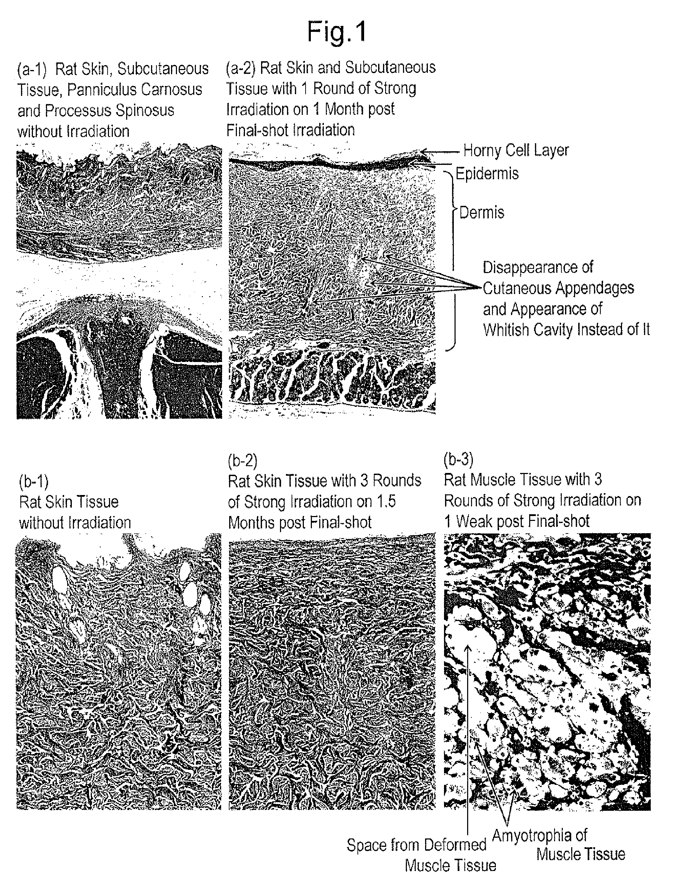

Fig. 1 is optical photomicrographs indicating the skin, the subcutaneous tissue including the panniculus carnosus and the processus spinosus, and the muscle tissue of the rats in the presence of near-infrared ray irradiation under different irradiation levels or in the absence of irradiation.

Fig. 1 (a-1) is an optical photomicrograph indicating the result of HE staining of the skin and the subcutaneous tissue including panniculus carnosus and the processus spinosus of the normal rats in the control group without near-infrared ray irradiation. As shown in the photomicrograph, the horny layers are thick and the epidermis is extremely rough, and a number of inter-spaces in the collagen fibers in the dermis can be observed. In deeper layers of the dermis, tunica dartos, areolar conductive tissue, periosteum and cortical bone can be observed, and on the both side of the processus spinosus, muscle can be observed. Bone-marrow cells densely stained violet can be observed inside the processus spinosus.

Fig. 1 (a-2) is an optical photomicrograph indicating the result of HE staining of the skin and the subcutaneous tissue of the rat at 1 month post final-shot at 40 J/cm² of 1 round of near-infrared ray irradiation. Decrease or disappearance of cutaneous appendages such as glandulae sebaceae, sudoriferous gland and hair root can be observed.

Fig. 1(b-1) is an optical photomicrograph indicating the result of AZAN staining of the skin tissue of the normal rats in the control group without near-infrared ray irradiation. As shown in the photomicrograph, the horny layer and the epidermis are extremely rough, and a number of cutaneous appendages such as glandulae sebaceae can be observed. Moreover, a number of inter-spaces in the collagen fibers in the dermis can be observed.

Fig. 1(b-2) is an optical photomicrograph indicating the result of AZAN staining of the rat skin tissue at 1.5 months post final-shot at 40 J/cm² of 3 rounds of near-infrared ray irradiation. As shown in the photomicrograph, the horny layers and the epidermis are smooth, and the wrinkles are disappeared. Also, extensive proliferation of small vessels in the dermis and extremely dense propagation of bundles of the collagen fibers in the upper dermis layers can be observed.

Fig. 1 (b-3) is an optical photomicrograph indicating the result of AZAN staining of the rat muscle tissue at 1 week post final-shot at 40 J/cm² of 3 rounds of near-infrared ray irradiation. As shown in the photomicrograph, amyotrophia and deformity of the muscle tissue can be observed.

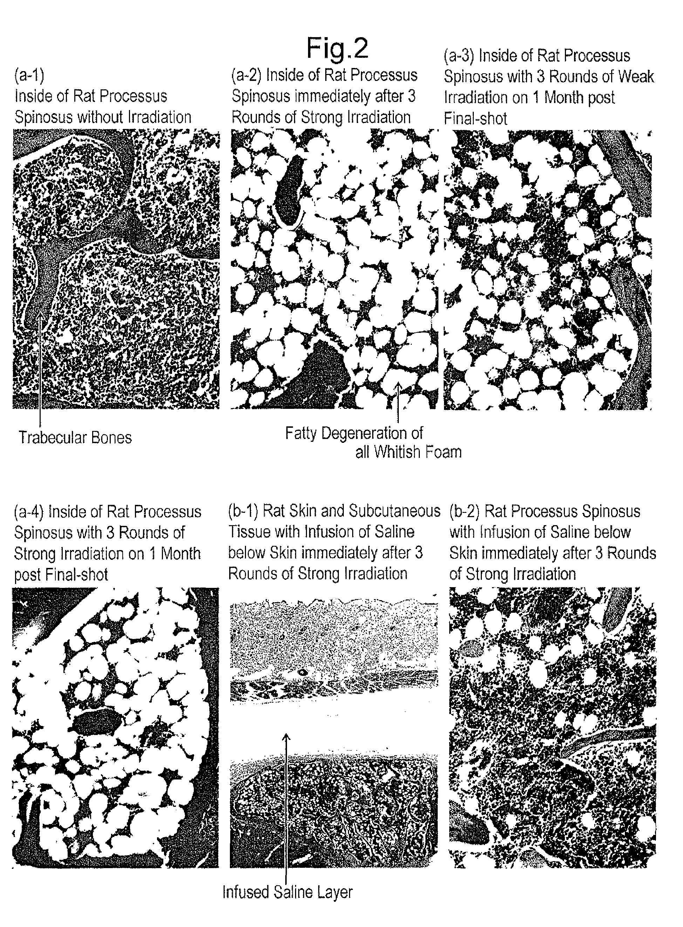

Fig. 2 is optical photomicrographs principally indicating the inside processus spinosus of the rat in the absence of the near-infrared ray irradiation or the presence of irradiation under different irradiation levels with or without subcutaneous injection of saline.

Fig. 2(a-1) is a welf-magnified optical photomicrograph indicating the result of HE staining of the inside of the processus spinosus of the normal rats in the control group without near-infrared ray irradiation. As shown in the photomicrograph, bone marrow cells densely stained violet and firm trabecular bones stained pink can be observed.

Fig. 2(a-2) is a well-magnified optical photomicrograph indicating the result of HE staining of the inside of the processus spinosus of the rat immediately after 3 rounds of near-infrared ray irradiation at 40 J/cm². As shown in the photomicrograph, decrease of the bone marrow cells densely stained violet and extent appearance of fatty degeneration which looks like whitish foam can be observed.

Fig. 2(a-3) is a well-magnified optical photomicrograph indicating the result of HE staining of the inside of the processus spinosus of the rat at 1 month post final-shot at 20 J/cm² of 3 rounds of near-infrared ray irradiation. As shown in the photomicrographs of (a-2) and (a-3), decrease of the bone marrow cells densely stained violet and appearance of the fatty degeneration which looks like whitish foam are slighter than those of near-infrared ray irradiation at 20 J/cm².

Fig. 2(a-4) is a well-magnified optical photomicrograph indicating the result of HE staining of the inside of the processus spinosus of the rat at 1 month post final-shot at 40 J/cm² of three rounds of near-infrared ray irradiation. As shown in the photomicrographs of (a-2) and (a-4), decrease of the bone marrow cells densely stained violet and extent appearance of the fatty degeneration which looks like whitish foam can be observed, and evident recovery thereof cannot be observed as compared with the processus spinosus immediately after the irradiation of the near-infrared rays.

Fig. 2(b-1) is an optical photomicrograph indicating the result of HE staining of the skin and the subcutaneous tissue with the panniculus carnosus and the processus spinosus of the rats with infusion of saline below the skin immediately after three rounds of near-infrared ray irradiation at 40 J/cm². As shown in the photomicrograph, decrease of the bone marrow cells densely stained violet and appearance of the fatty degeneration which looks like whitish foam are evidently slighter than those without injection of the saline under the near-infrared rays irradiation. It is inferred that the damage of deeper layer tissue than the skin is suppressed minimumly, because the near-infrared rays are easily absorbed by water.

Fig. 2(b-2) is a well-magnified optical photomicrograph which indicates the result by the HE staining of inside of the processus spinosus of the rat with the infusion of saline below the skin immediately after three shots of the near-infrared rays irradiated at 40 J/cm² to the rat As shown in the photomicrograph, it is obvious that decrease of the bone marrow cells stained to violet very densely and appearance of the fatty degeneration which looks like whitish foam are evidently slighter than them without injection of saline under the near-infrared rays irradiation.

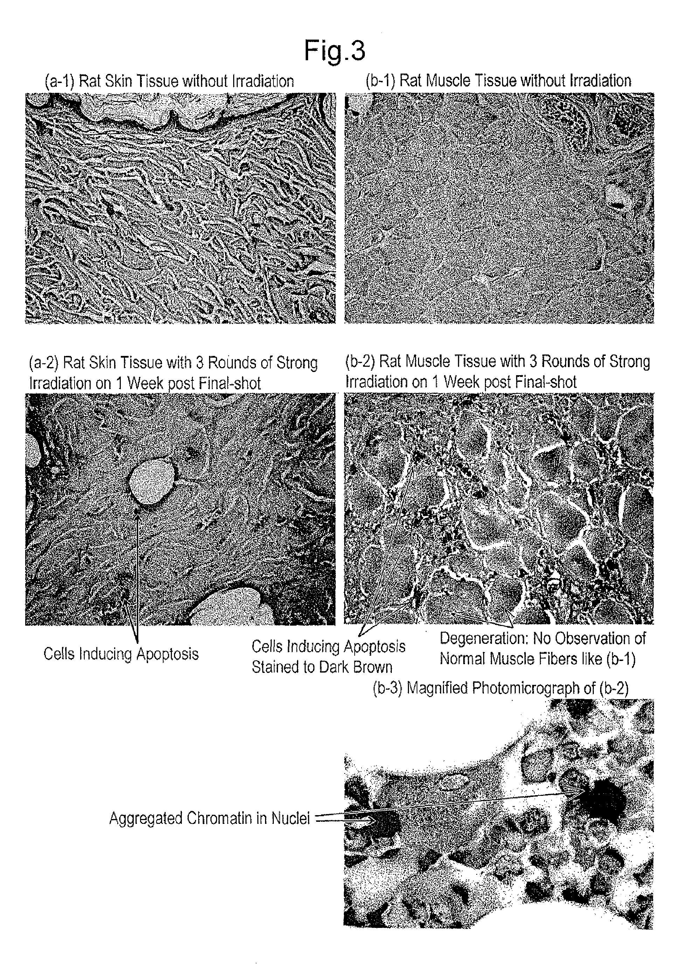

Fig. 3 is optical photomicrographs indicating tissue of skin and the muscle of the rats in cases with the irradiation of the near-infrared rays or without irradiation.

Fig. 3(a-1) is an optical photomicrograph which indicates the result by the TUNEL staining technique of the skin tissue of the normal rats in the control group without irradiation of the near-infrared rays. As shown in the photomicrograph, no cells inducing apoptosis in the horny layer or the epidermis or the dermis are evidently observed.

Fig. 3(b-1) is an optical photomicrograph which indicates the result by the TUNEL staining technique of the muscle tissue of the normal rats in the control group without irradiation of the near-infrared rays. As shown in the photomicrograph, no cells inducing apoptosis in the panniculus carnosus are evidently observed.

Fig. 3(a-2) is an optical photomicrograph which indicates the result by the TUNEL staining technique of the skin tissue of the rat after a lapse of 1 week of three shots of the near-infrared rays irradiated at 40 J/cm² to the rat. As shown in the photomicrograph, cells inducing apoptosis in the dermis are evidently observed.

Fig. 3(b-2) is an optical photomicrograph which indicates the result by the TUNEL staining technique of the muscle tissue of the rat after a lapse of 1 week of three shots of the near-infrared rays irradiated at 40 J/cm² to the rat. As shown in the photomicrograph, a lot of cells inducing apoptosis as well as amyotrophia and deformity of the widely muscle tissue are very evidently observed.

Fig. 3(b-3) is a well-magnified optical photomicrograph of 1000-fold magnifications (i.e. maximum magnifications of the optical microscope) which indicates the result by the TUNEL staining technique of the muscle tissue of the rat after a lapse of 1 week of three shots of the near-infrared rays irradiated at 40 J/cm² to the rat. As shown in the photomicrograph, it is obvious that chromatin in nuclei is aggregated and apoptosis is induced thereby.

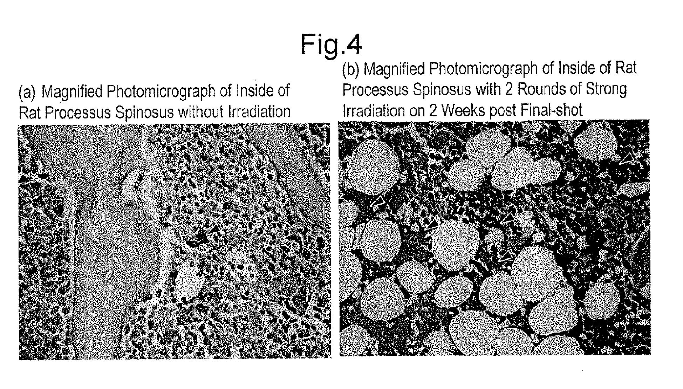

Fig. 4 is magnified optical photomicrographs indicating the inside of processus spinosus of the rat in cases with the irradiation of the near-infrared rays or without irradiation.

Fig. 4(a) is a well-magnified optical photomicrograph which indicates the result by the TUNEL staining technique of inside of the processus spinosus of the normal rats in the control group without irradiation of the near-infrared rays. As shown in the photomicrograph, a few of the bone marrow cells inducing apoptosis are observed (indicated by arrows thereof).

Fig. 4(b) is a well-magnified optical photomicrograph which indicates the result by the TUNEL staining technique of the inside of the processus spinosus of the rat after a lapse of 3 weeks of two shots of the near-infrared rays irradiated at 40 J/cm² to the rat. As shown in the photomicrograph, and fatty degeneration which looks like whitish foam appears widely and a lot of bone marrow cells inducing apoptosis in the processus spinosus are evidently observed (indicated by arrows thereof).

The above-mentioned histological investigations show medically, histologically and objectively that the cutaneous appendages such as the glandulae sebaceae, the glandulae sudoriferae and the hair root are decreased or disappeared due to exposure with the near-infrared rays, and also exposure with high output power and continuous irradiation cause atrophy, degeneration and apotosis of muscle cells. When the skin is exposed with sunrays for a long period of time, not only the ultraviolet rays damage the skin but also the near-infrared rays penetrated through the skin cause imperceptibly serious damage on various human tissue such as the skin, subcutaneous tissue, muscle, internal organs, bone and brain, and this damage caused by the near-infrared rays accelerates aging in one way.

This supports the clinical results that the skin of the person who does sunbathing frequently ages faster, and atrophy of such person's dermis and subcutaneous tissue is significant. Also, this can be the explanation of that those people who are exposed with sunrays for a long time for long periods, for example those people living in high altitude, have relatively thicker skin, especially dermis, to protect themselves against the near-infrared ray. It can be also explained that those people living in high altitude have sunburn-puffy face as their dermis are exposed with the near-infrared rays and thus heated resulting edema by burn injury. On the other hand, because the skin of those people with thin and fair skin, for example the Caucasians, cannot fully absorb the near-infrared rays with the skin and their muscle is thin, their skin and subcutaneous tissue tend to sag as aging, and this result the fact that the Caucasians receive a face lift of anti-aging operation way more than non-Caucasians do. Although a large number of people try to prevent the ultraviolet rays, blocking the ultraviolet rays does not keep the skin and the body from aging as well as cutaneous cancer and dermatological disease.

According to the results, a W/O type external medication for the skin as the protective agent against damage of biological tissue caused by near-infrared rays and a pair of glasses as the product for protecting biological tissue against damage caused by the near-infrared rays using the external medications were prepared as following Examples.

### (Example 1) Trial preparation of the external medication for the skin intended to block the near-infrared rays

The creamy external medication for skin was prepared by kneading the following ingredients;
titanium oxide (10 weight%) and zinc oxide (5 weight%) as the near-infrared ray penetration blocker,
decamethylcyclopentanesiloxane (26 weight%),
1,3-butylene glycol (10 weight%),
methylpolysiloxane (5 weight%),
polyglyceryl-3-polydimethylsiloxyethyl dimethicone (4 weight%),
phenoxyethanol (0.4 weight%), and
purified water (the rest thereof).
This external medication for skin has approximately 25 of a sun protection factor (SPF) as a calculated value.

### (Example 2) Another trial preparation of the external medication for the skin intended to block the near-infrared rays

The creamy external medication for skin was prepared by kneading the following ingredients;
titanium oxide (5 weight%) and zinc oxide (20 weight%) as the near-infrared ray penetration blocker,
Decamethylcyclopentanesiloxane (29 weight%),
1,3-butylene glycol (8 weight%),
methylpolysiloxane (2 weight%),
Polyglyceryl-3-polydimethylsiloxyethyl dimethicone (4 weight%),
phenoxyethanol (0.4 weight%), and
purified water (the rest thereof).
This external medication for skin has approximately 45 of a sun protection factor (SPF) as a calculated value.

### (Example 3) The other trial preparation of the external medication for the skin intended to block the near-infrared rays

The creamy external medication for skin was prepared by kneading the following ingredients;
titanium oxide (7 weight%) and zinc oxide (15 weight%) as the near-infrared ray penetration blocker,
decamethylcyclopenfanesiloxane (29 weight%),
1,3-butylene glycol (8 weight%),
methylpolysiloxane (2 weight%),
polyglyceryl-3-polydimethylsiloxyethyl dimethicone (4 weight%),
phenoxyethanol (0.4 weight%), and
purified water (the rest thereof).
This external medication for skin has approximately 38 of a sun protection factor (SPF) as a calculated value.

### (Example 4) Other trial preparation of the external medication for the skin intended to block the near-infrared rays

The creamy external medication for skin was prepared by kneading the following ingredients;
titanium oxide (3 weight%) and zinc oxide (10 weight%) as the near-infrared ray penetration blocker,
decamethylcyclopentanesiloxane (42 weight%),
1,3-butylene glycol (8 weight%),
methylpolysiloxane (2 weight%),
polyglyceryl-3-polydimethylsiloxyethyl dimethicone (2 weight%),
phenoxyethanol (0.4 weight%), and
purified water (the rest thereof).

This external medication for skin has approximately 22 of a sun protection factor (SPF) as a calculated value.

The titanium oxide in above Examples 1-4 has 10-20 nm of primary particle size, 10-20 nm of transverse diameter and 50-70 nm of longitudinal diameter, and is in the shape of spindle. And the zinc oxide in above Examples 1-4 has 20-30 nm of primary particle size.

### (Comparative Example) Trial preparation of the external medication for the skin intended to comparison

A creamy external medication for skin was prepared by kneading the following ingredients;
decamethylcyclopentanesiloxane (38 weight%),
1,3-butylene glycol (10 weight%),
methylpolysiloxane (7 weight%),
phenoxyethanol (0.4 weight%), and
purified water (the rest thereof).

Physical and chemical analyses are performed to appreciate the performance of the external medications in Examples 1-4 and Comparative Example.

### (Determination of Transmittance)

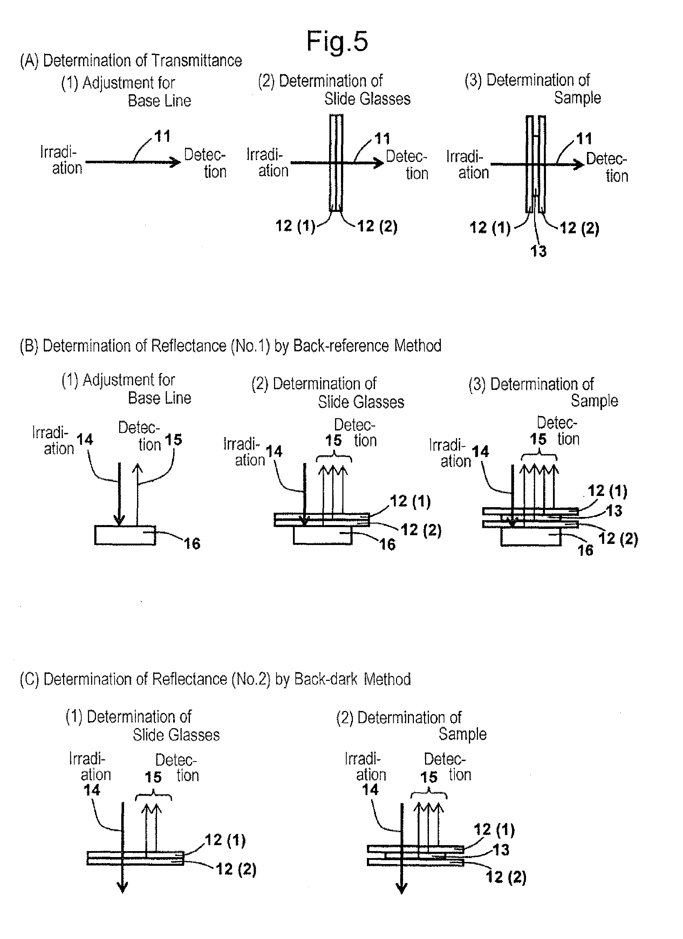

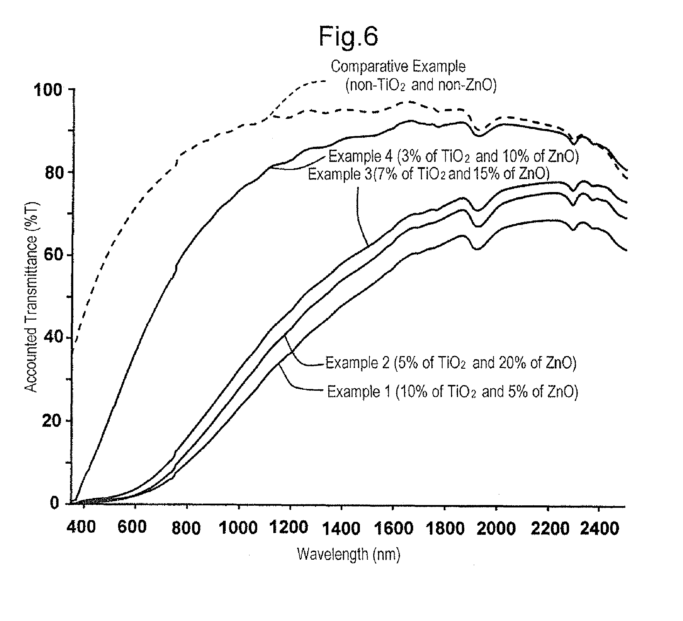

Transmittance was determined by using ultraviolet/ visible light/near-infrared spectrophotometer: V-570, which is available from JASCO Corporation. The conditions are as follows; determining range is from 190 to 2500 nm, band width is 5 nm under visible region and is 20 nm under near-infrared region, sampling intervals of datum is 2 nm, and scanning speed is 1000nm/min. Slide glasses for near-infrared rays were used for determination thereof. Minutely to explain with referring a method for the determination of the transmittance indicated by Fig. 5 (A), details of the determination thereof are as follows. Initially, the transmittance of blank in all wavelength of the determining range under the air atmosphere is determined, and a base line for the transmittance is adjusted. And a pair of slide glasses 12 (1) and (2) is put together, then light 11 having the wave length of the determining range were irradiated to the glasses. Transmitted light 11 was detected by a spectroscope of the spectrophotometer to determine the transmittance of the glasses 12 (1) and (2) as a reference. Additionally, an external medication for skin as a sample 13 was sandwiched between both slide glasses 12 (1) and (2), and the transmittance of the external medication for the skin was determined similarly by using the spectrophotometer. In Fig. 6, converted transmittances (i.e. %T) of the external medications for the skin are shown, when the transmittances under all wavelength of the determining range of only the slide glasses are accounted as 100 %. Incidentally, datum below 350 nm is not indicated, because the transmittance of the slide glasses 12 (1) and (2) under ultraviolet region is impossible to be determined.

As regards with the determination of the transmittance, it is seemed that the lower the transmittance of the near-infrared rays of the external medication for the skin, the higher the absorptance and reflectance thereof. As shown in Fig. 6, the transmission of the near-infrared rays of approximately 770-2500 of the external medications including titanium oxide and zinc oxide as the near-infrared ray penetration blocker in Examples 1-4 is lower significantly than the transmission of the near-infrared rays of the external medications without the blocker in Comparative Examples. Especially it is remarkably obvious that the lower the wavelength the lower the transmittance in Examples. Particularly, the transmittance of the external medication for the skin in Example 1 is decreased at the rate of approximately 70% at the maximum than the transmittance of the external medication for the skin in Comparative Example. So it is seemed that the absorptance and reflectance of the near-infrared rays in Examples are higher than them of Comparative Example. Therefore the external medications of Examples are effective for the near-infrared ray penetration blocker.

### (Determination of Reflectance by Back-reference Method)

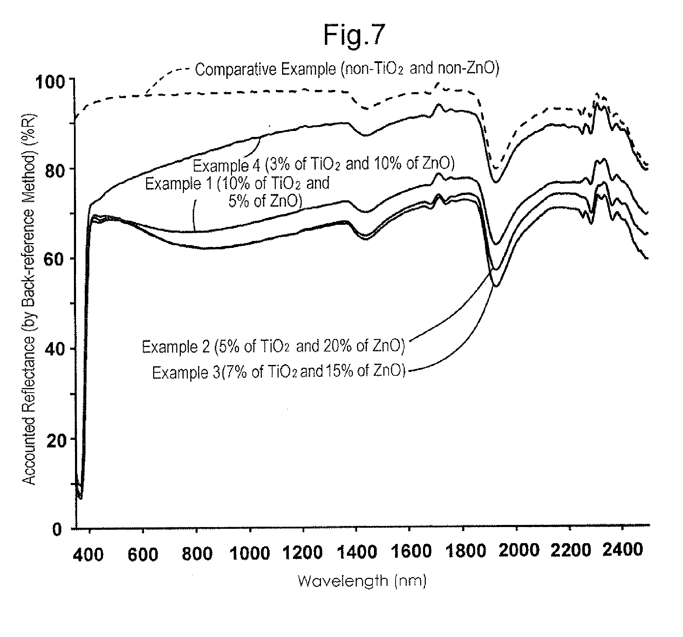

Reflectance was determined by using ultraviolet/ visible light/near-infrared spectrophotometer: V-570, which is available from JASCO Corporation. The conditions are as follows; reflective reference plates attached to the spectrophotometer are used, determining range is from 190 to 2500 nm, band width is 5 nm under visible region and is 20 nm under near-infrared region, sampling intervals of datum is 2 nm, and scanning speed is 1000nm/min. Slide glasses for near-infrared rays were used for determination thereof. Minutely to explain with referring a method for the determination of the reflectance (i.e. a back-reference method) indicated by Fig. 5 (B), details of the determination thereof are as follows. Initially, the reflectance of the reflective reference plate 16 in all wavelength of the determining range is determined, and a base line for the reflectance is adjusted. And a pair of slide glasses 12(1) and (2) is put together, then light 14 having the wave length of the determining range were irradiated to the glasses. Reflected light 15 was detected to determine the reflectance of both slide glasses 12 (1) and (2) as a reference. Additionally, an external medication for skin as a sample 13 was sandwiched between both slide glasses 12 (1) and (2), and the reflectance of the external medication for the skin was determined similarly by using the spectrophotometer. The light transmitted though both glasses 12 (1) and (2) and the external medication for skin as the sample 13 was reflected on the reference plate 16. In Fig. 7, converted reflectance (i.e. %R) of the external medication for the skin is shown, when the reflectance under all wavelength of the determining range of only the slide glasses 12 (1) and (2) are accounted as 100%.

As regards with the determination of the reflectance by the back-reference method, it is seemed that the lower the reflectance of the near-infrared rays of the external medication for the skin, the higher the absorptance thereof, because the light transmitted through the external medication for the skin including the near-infrared ray penetration blocker are reflected on the reference plate.

According to the reflectance by the back-reference method, intensity of absorption of the near-infrared rays into the external medication for the skin including the near-infrared ray penetration blocker is determined. As shown in Fig. 7, the reflectance of the near-infrared rays of approximately 400-2500 by the external medications including titanium oxide and zinc oxide as the near-infrared ray penetration blocker in Examples 1-4 is lower significantly than the reflectance of the near-infrared rays by the external medications including no near-infrared ray penetration blocker in Comparative Example. It is seemed that the lower the reflectance of the near-infrared rays of the external medication for the skin, the higher the absorptance thereof. Therefore it is obvious that properties of blocking the near-infrared ray penetration are excellent.

### (Determination of Reflectance by Back-dark Method)

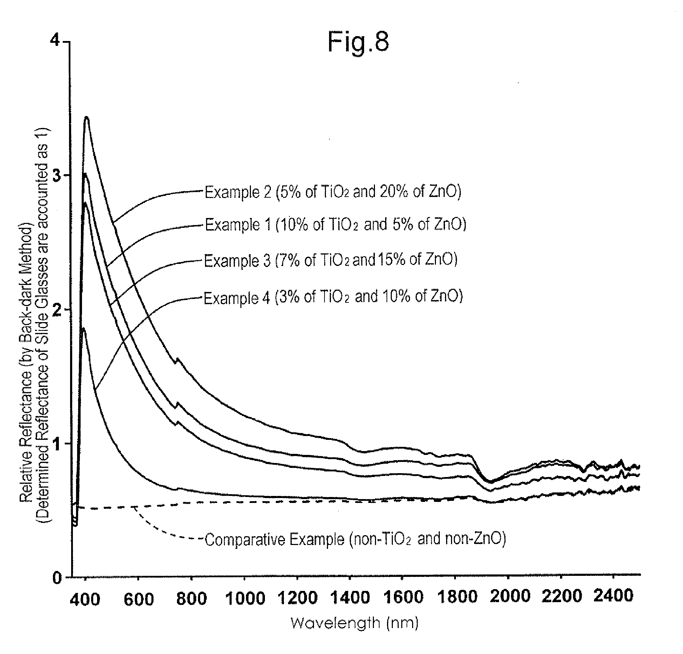

Reflectance was determined by using ultraviolet/ visible light/near-infrared spectrophotometer: V-570, which is available from JASCO Corporation. The conditions are as follows; determining range is from 190 to 2500 nm, band width is 5 nm under visible region and is 20 nm under near-infrared region, sampling intervals of datum is 2 nm, and scanning speed is 1000nm/min. Slide glasses for near-infrared rays were used for determination thereof. Minutely to explain with referring a method for the determination of the reflectance (i.e. a back-dark method) indicated by Fig. 5 (C), details of the determination thereof are as follows. Initially, a pair of slide glasses 12 (1) and (2) is put together, then light 14 having the wave length of the determining range was irradiated to the glasses. Reflected light 15 was detected to determine the reflectance of the slide glasses 12 (1) and (2) as a reference. Additionally, an external medication for skin as a sample 13 was sandwiched between both slide glasses 12 (1) and (2), and the reflectance of the external medication for the skin was determined similarly by using the spectrophotometer. Any light transmitted through slide glasses 12 (1) and (2) or the external medication for skin as the sample 13 was not reflected to be detected by a spectroscope of the spectrophotometer. In Fig. 8, relative reflectances of the external medication for the skin of Examples 1-4 and Comparative Example is shown, when the reflectances under all wavelength of the determining range of only the slide glasses 12 (1) and (2) are accounted as 1.

As regards with the determination of the reflectance by the back-dark method, it is seemed that the higher the relative reflectance of the back-dark reflection the higher the reflectance of the near-infrared rays thereof, because the light transmitted through the external medication for the skin including the near-infrared ray penetration blocker was not reflected to be detected. According to the reflectance by the back-dark method, intensity of reflection of the near-infrared rays by the near-infrared ray penetration blocker is determined. As shown in Fig. 8, the relative value of the back-dark reflectance under the near-infrared rays of approximately 400-2500 by the external medications including titanium oxide and zinc oxide as the near-infrared ray penetration blocker in Examples 1-4 is lower significantly than the reflectance of the near-infrared rays of the external medications including no near-infrared ray penetration blocker in Comparative Example. It is seemed that the higher the relative value of the external medication for the skin the higher the absorptance of the near-infrared rays, therefore it is obvious that properties of blocking the near-infrared ray penetration of the external medication for the skin including the near-infrared ray penetration blocker in Examples are excellent.

As above-mentioned, the external medications of Examples 1 and 2 among Examples have most sufficient reflection and absorption of the near-infrared rays, and is excellent thereby.

### (Example 5) Other trial preparation of the external medication for the skin intended to block the near-infrared rays

The following ingredients are used under below weight ratios. [powdery ingredients]
1. titanium oxide dispersion: Solavell CT-1 00 being available from CRODA, in which contents thereof are alkyl benzoate having 12-15 carbons of the alkyl, titanium oxide, polyhydroxystearic acid, aluminum stearate and alumina (25% as the converted ratio of titanium oxide
2. zinc oxide (5%)
3. cetyl dimethicone copolyol: ABIL EM90 which is available fromDegussa (1%)
4. PEG-10 dimethicones: SS2910 which is available from Dow Corning Toray Co.,Ltd. (1 %)
5. phenyl trimethicone: SH556; which is available from Dow Corning Toray Co.,Ltd. (1 %)
   [oily phase materials]
6. cyclopentasiloxane: SH245; which is available from Dow Corning Toray Co.,Ltd. (1 %)
7. trimethylsiloxysilicate: BY11-018; which is available from Dow Corning Toray Co.,Ltd. (1 %)
   [aqueous ingredients]
8. glycerin (1%)
9. butylene glycol (i.e. BG, 10 %)
10. phenoxyethanol (0.3 %)
11. purified water (the rest thereof)

The creamy external medication for skin was prepared by kneading the above ingredients.

The external medication for the skin was independently applied twice onto one side of face and arm of 10 normal persons having no dermatological disease, and then they sunbathed under the sunshine for 1 hour. Potencies of the external medication for the skin were evaluated by comparing the applied side of the face and the arm with the non-applied side of the face and the arm. While sensations of burning or warmth were observed in all of the non-applied side of the face and arm and in 7 of them sunburn was observed, findings such as sensations of burning or warmth, sunburn, erythema, tumentia and vesicle that were caused due to the exposed near-infrared rays were not observed in the applied side face and arm of all persons.

### (Example 6) Trial preparation of the external medication for the skin intended to block the near-infrared rays

A creamy external medication for skin was prepared as same as Example 5 by using 20 % of converted content of titanium oxide in dispersing element and 15% of zinc oxide instead of using the titanium oxide and the zinc oxide in Example 5. Potencies thereof were as well as the external medication of Example 5.

When the external medication for the skin was applied onto rats and the near-infrared rays were irradiated to the rats as well as the above-mentioned histological investigation, no abnormality was occurred as well as the case without irradiation thereof. The external medication for the skin prevents the damage, which is caused by exposure of the near-infrared rays, in tissue of the skin, subcutaneous tissue or deeper tissue than skin, such as horny layer, epidermis, dermis, panniculus carnosus, collagen fiber of the dermis, cutaneous appendage illustrated by processus spinosus, bone marrow cells, and trabecular bones. Therefore they were maintained to be kept normal. Simultaneously other damages caused by exposure of ultraviolet rays are also prevented.

### (Example 7) Trial preparation of eyeglasses intended to block the near-infrared rays

As a film shielding against the near-infrared rays, Eco Shield FILM, which is available from INTERCEPT Co., Ltd., was used. The film includes the above-mentioned indium oxide doped with tin as a metal-oxide semiconductor which is capable to shield against the near-infrared rays. Eyeglasses were prepared by sticking the film shielding against the near-infrared rays on plano-lens made from glass or plastics. On the other hand, plano eyeglasses sticking no film was used as reference.

Difference of development of asthenopia between using eyeglasses with or without the film was evaluated. 10 normal persons having no previous ophthalmologic disease, no visual impairment and no visual field disturbance wore the eyeglasses respectively. They looked straightly the sun out of doors. And levels of the asthenopia thereby were investigated and evaluated. Incidentally if photophobia thereof was too strong, they did not look straightly the sun and looked neighborhood of the sun. They looked the same viewing location in either case using the glasses with or without the film. The asthenopia was evidently relieved in all case using the glasses with the film in comparison with the case using the glassed without the film.

Other embodiments using the aforesaid organic compounds or inorganic compounds are successful as well as the above-mentioned embodiments using titanium oxide, zinc oxide or indium oxide as the near-infrared ray penetration blocker in Examples 1-7. The protective agent against damage of biological tissue caused by near-infrared rays comprises other additives which are mentioned above.

### Industrial Applicability

The present protective agent against damage of biological tissue caused by near-infrared rays can be used for daily-use items that people wear, touch or have, and is useful for protect the skin and the deeper tissue than the skin from damage by the near-infrared rays that is radiated to the skin daily from sunrays or thermal source. Also, the protective agent can be used for investigating a mechanism of pathogenesis or development of disease or disorder caused by the near-infrared rays. It can be used in the form of preventive medicine or therapeutic medicine for prevention or treatment.

The daily-use product for protecting biological tissue against damage caused by near-infrared rays prevents the damage and aging of body tissue as well as pathogenesis and development of various disease and disorder caused by the near-infrared rays. As a consequence, such product contributes to healthcare economic reconstruction. Also, such products are useful for reducing frequency of operations or medications performed for treatment and physical load to patients of various disease or disorder caused by the near-infrared rays.

## Claims

1. A protective agent against damage of biological tissue caused by near-infrared rays comprising;
a near-infrared ray penetration blocker that absorbs, reflects or scatters the near-infrared ray to prevent the damage of skin and deeper tissue than the skin in a living body caused by exposure with the near-infrared rays.

2. The protective agent according to claim 1, wherein the near-infrared ray penetration blocker is at least one selected from the group consisting of metal powder; metal-oxide powder; metal-oxide leaves; metal carbide; metal nitride; metal boride; metal powder coated with metal nitride, metal boride or metal carbide; resin powder coated with metal oxide; mineral powder; ceramics; resin; onium salt compound; azo compound; anthraquinone compound; anfra compound; cyanine compound; metal complex compound; squarylium compound; naphthalocyanine comopound; phthalocyanine compound; polyene compound; and polymethine compound.

3. The protective agent according to claim 1, wherein a content of the near-infrared ray penetration blocker ranges from 3 to 45 weight %.

4. The protective agent according to claim 3, wherein the near-infrared ray penetration blocker is the metal-oxide powder consisting of titanium oxide powder and zinc oxide powder.

5. The protective agent according to claim 4, wherein a content of the titanium oxide powder ranges from 3 to 25 weight % and a content of the zinc oxide powder ranges from 5 to 20 weight %.

6. A product for protecting biological tissue from damage caused by near-infrared rays comprising;
a protective agent against damage of biological tissue by a near-infrared ray comprising a near-infrared ray penetration blocker that absorbs, reflects, or scatters the near-infrared ray to prevent the damage of skin and a deeper tissue than the skin in a living body exposed with the near-infrared ray,
wherein the protective agent is applied, absorbed, adhered, attached or contained to at least a part of a base material, or is contained into the medium in chemicals.

7. The product according to claim 6, wherein a content of the protective agent ranges from 0.0001 to 99.9 % by weight to the base material or the chemicals.

8. The product according to claim 6, wherein the base material is paper, fabric, nonwoven fabric, a wood plate, leather, a resin film, a resin sheet, a resin plate, a glass plate and/or a metal plate, and the medium is water, an organic solvent, gel, sol, powder and/or binder.

9. The product according to claim 6,
which comprises the base material and used for clothes, furnishing goods, a protector, accessories, a filter, building materials, external materials, interior materials or decoration materials, or
which is the chemicals and used for cosmetics, external medicine for skin, or coating materials.

10. The product according to claim 6, which is used for preventing aging of the living body or the tissue, morphological variation thereof, functional deterioration thereof, disease pathogenesis, or disease ingravescence.
